# EUROPEAN PATENT SPECIFICATION

(11) **EP 3 019 599 B1**
(45) Date of publication and mention of the grant of the patent: **13.06.2018**
(21) Application number: 14822296.1
(22) Date of filing: 10.07.2014
(51) Int. Cl.: C12N 5/0775, A61K 35/36, A61K 35/12

(54) **METHOD FOR ISOLATING STROMAL VASCULAR FRACTION**
VERFAHREN ZUR ISOLIERUNG EINES STROMAGEFÄSSABSCHNITTS
MÉTHODE D'ISOLEMENT DE FRACTION STROMA-VASCULAIRE

(30) Priority: 10.07.2013 SG 201305309
(43) Date of publication of application: 18.05.2016
(73) Proprietor: Agency for Science, Technology and Research, Singapore 138632 (SG)
(72) Inventor: SUGII, Shigeki, Singapore 138667 (SG); ONG, Wee Kiat, Singapore 138667 (SG)
(74) Representative: Herzog, Fiesser & Partner Patentanwälte PartG mbB
(86) International application number: PCT/SG2014/000327
(87) International publication number: WO 2015/005871

(56) References cited:
- WO-A1-03/024215
- WO-A1-2014/036094
- US-A1- 2014 017 783
- BIANCHI, F. ET AL.: 'A new nonenzymatic method and device to obtain a fat tissue derivative highly enriched in pericyte-like elements by mild mechanical forces from human lipoaspirates' CELL TRANSPLANTATION. vol. 22, 2013, pages 2063 - 2077, XP002752470
- SHAH, FS. ET AL.: 'A non-enzymatic method for isolating human adipose tissue-derived stromal stem cells''.' CYTOTHERAPY. vol. 15, 2013, pages 979 - 985, XP055299178
- BAPTISTA, LS. ET AL.: 'An alternative method for the isolation of mesenchymal stromal cells derived from lipoaspirate samples' CYTOTHERAPY vol. 11, 2009, pages 706 - 715, XP009167618
- YOSHIMURA, K. ET AL.: 'Characterization of freshly isolated and cultured cells derived from the fatty and fluid portions of liposuction aspirates' J. CELL. PHYSIOL. vol. 208, 2006, pages 64 - 76, XP002498508
- KIM, JB. ET AL.: 'Cryopreservation of human adipose tissues using human plasma' J. BIOTECHNOL. BIOMATERIAL vol. 1, 2011, page 107, XP055308801
- WELCH, ZR. ET AL.: 'Isolating adipose-derived stromal cells through mechanical disruption and citrate phosphate dextrose' 56TH ANNUAL MEETING OF THE ORTHOPAEDIC RESEARCH SOCIETY March 2010, NEW ORLEANS., XP008182719 Retrieved from the Internet: <URL:http://www.ors.org/Transactions/56/072 7.pdf> [retrieved on 2014-09-21]
- BUSSER, H. ET AL.: 'Isolation of adipose-derived stromal cells without enzymatic treatment: expansion, phenotypical, and functional characterization' STEM CELLS AND DEVELOPMENT vol. 23, 2014, pages 2390 - 2400, XP055308802

## Description

### FIELD OF THE INVENTION

The present invention relates to the field of molecular biology. In particular, the present invention relates to an apparatus for use in cell isolation.

### BACKGROUND OF THE INVENTION

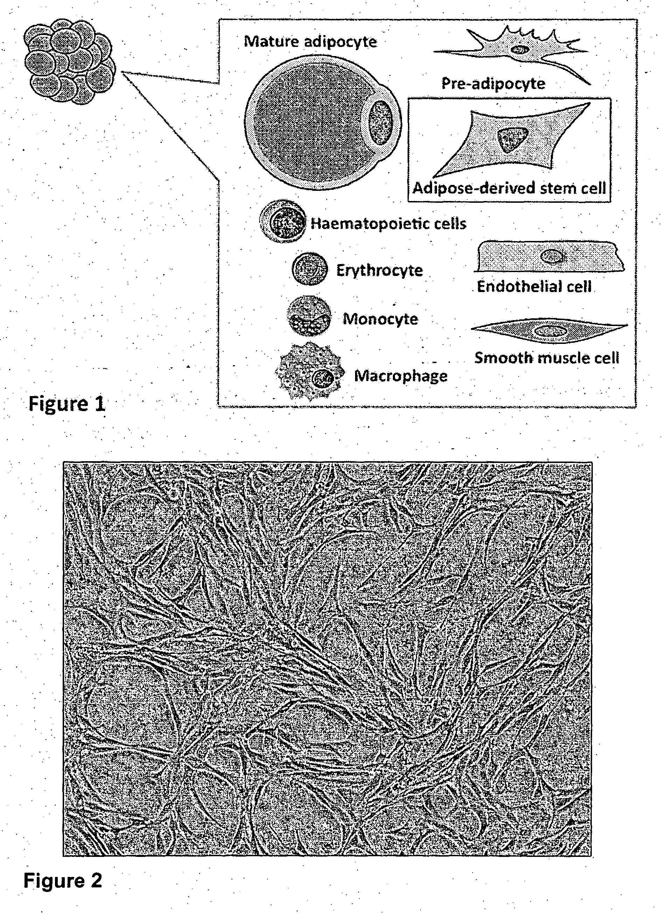

Stem cells are ideal sources for developing regenerative therapeutics. Typically, stem cells present in the human body are limited in number, and protocols to fully optimize their efficiency of purification and use are still under development. Fat tissue or adipose tissue has increasingly been recognized as a promising source of stem cells that are potentially beneficial for regenerative medicine. In addition to mature adipocytes, adipose tissue contains relatively abundant progenitor and mesenchymal stem cell (MSC) populations in the stromal vascular fraction (SVF) (see Figure 1). It is estimated that as many as 1% of stromal vascular fraction cells are mesenchymal stem cells, which in the present disclosure also called adipose-derived stem cells (ASCs). This is in contrast to mesenchymal stem cells representing only 0.001-00.2% in the bone marrow, which is considered a standard hub of adult stem cells (Fraser *et al.* 2006).

In view of the rise in obesity in Singapore and worldwide, fat tissue pose as a unique reservoir of stem cells that is available in abundance and is relatively easy to obtain. Liposuction, a procedure to remove excess fat tissue, is among the most common plastic surgeries performed in the developed countries. For example, it is known that there are over 313,000 liposuction operations in the USA in 2012. Additionally, in contrast to bone marrow-derived mesenchymal stem cells that require initial plating with high density (more than 50,000 cells per cm²), adipose-derived stem cells can be seeded at a density as low as 3,000 cells per cm² for subsequent growth in culture (Sotiropoulou, P.A., *et al.* 2006). Adipose-derived stem cells and bone marrow-derived mesenchymal stem cells are proliferative and multipotent, having the capacity to differentiate into limited cell types such as cells of fat, bone, cartilage and muscle. In addition, adipose-derived stem cells were shown to differentiate into pancreatic endocrine cells, neurons, epithelia and endothelia, which would be useful for tissue and cell replacement therapies (Cawthorn, *et al.,* 2012; Gimble, *et al.,* 2007 and Ong, W.K. and S. Sugii, 2013). Adipose-derived stem cells can also potentially be used for tissue and wound repair because they intrinsically secrete immunomodulatory, angiogenic, anti-apoptotic and hematopoietic factors. There are currently more than 80 ongoing clinical trials using stromal vascular fraction cells or adipose-derived stem cells worldwide in a wide range of disease areas (Lim, *et al.,* 2014).

It has also been demonstrated that adipose-derived stem cells are also ideal for "reprogramming" into induced pluripotent stem (iPS) cells (Sugii, S. *et al.,* 2011; Sugii, S. *et al.,* 2010). The reprogramming efficiencies of adipose-derived stem cells are substantially higher than those reported for human fibroblasts (up to 100 fold) and the process can be performed without the requirement of an animal feeder cells (WO2011/032025A2). With the establishment of feeder-free, xeno-free protocols to generate adipose-derived stem cells-derived iPS cells, this opens up even wider possibilities in the applications of fat tissues, especially when iPS cells, like embryonic stem cells, are theoretically capable of becoming any cell types of a human or animal body.

Despite potentials for adipose-derived stem cells in cell replacement and regenerative therapies, technologies to process and develop fat tissue-derived products are still at an early stage. A typical protocol of adipose-derived stem cell isolation involves digestion of isolated adipose tissue by enzyme collagenase, followed by centrifugation to separate floating adipocytes and adipose-derived stem cells-containing stromal vascular fraction in the pellet fraction (Lim, *et al.,* 2014). A few companies have also developed semi-automated devices that process fat tissue and isolate stromal vascular fraction, which involves closed systems with disposables that digest adipose tissue harvested by liposuction into cells, separate and concentrate them into suspension for immediate delivery into patients (Lim, *et al.,* 2014). Currently, most methods known in the art mainly use collagenase or other dissociating enzymes for digestion. However, use of enzymes such as collagenase can be a safety concern as collagenase is commonly produced from gram-positive bacteria *Clostridium histolyticum.* Use of enzyme also makes quality control difficult because it has shelf-life and lot-to-lot variability in its activity. In addition, collagenase digestion requires long processing time (around one hour) and often results in heterogeneity in yield in cell number. In view of the use of bacterially-derived collagenase, the process also necessitates extensive washing that leads to increasing total time and costs involved in the isolation or recovery process. Additionally, processes as known in the art also require a significant amount of manual handling by technicians that increases the risks of contamination.

In view of the problems encountered in the art, there is a need to provide an alternative method for recovering stromal vascular fraction from a sample.

### SUMMARY OF THE INVENTION

In one aspect, there is provided a method for recovering stromal vascular fraction from a sample. In one example, the method comprises providing a mechanical force capable of breaking the sample into a single cell suspension whilst maintaining intact stromal vascular fraction cells' cellular structures, wherein the mechanical force is mincing and/or homogenization, wherein the method is a non-enzymatic digestion.

Also described herein is a method of treating a subject in need of cell therapy. In one example, the method of treating the subject of this aspect comprises administering the cells of the stromal vascular fraction obtained from the method of the present disclosure to the subject in need thereof.

Also described herein is a method of treating a subject in need of cell therapy. In one example, the method of treating the subject comprises administering the adipose-derived stem cells obtained from the method of the present disclosure to the subject in need of cell therapy.

### BRIEF DESCRIPTION OF THE DRAWINGS

The invention will be better understood with reference to the detailed description when considered in conjunction with the non-limiting examples and the accompanying drawings, in which:
**Figure 1** shows an illustration of the types of cells that can be found in stromal vascular fraction. Figure 1 provides a schematic illustration of the types of cells that can be recovered by the method of the present disclosure.
**Figure 2** shows a microscopic image of cellular growth and morphology after isolation using typical enzymatic method known in the art and culturing to enrich adipose-derived stem cells.
**Figure 3** shows a microscopic image of isolated cell samples (at day 4 of culture; at 100x magnification) as obtained by methods known in the art (i.e. by enzyme method) at day 4 of culture.
**Figure 4** shows a microscopic image of cell samples (at day 4 of culture; at 100x magnification) after incubation at 37°C for 1 hour followed by homogenizing for 65 seconds. Figure 4 shows the microscopic image of cellular morphology of cells recovered using one method of the present disclosure.

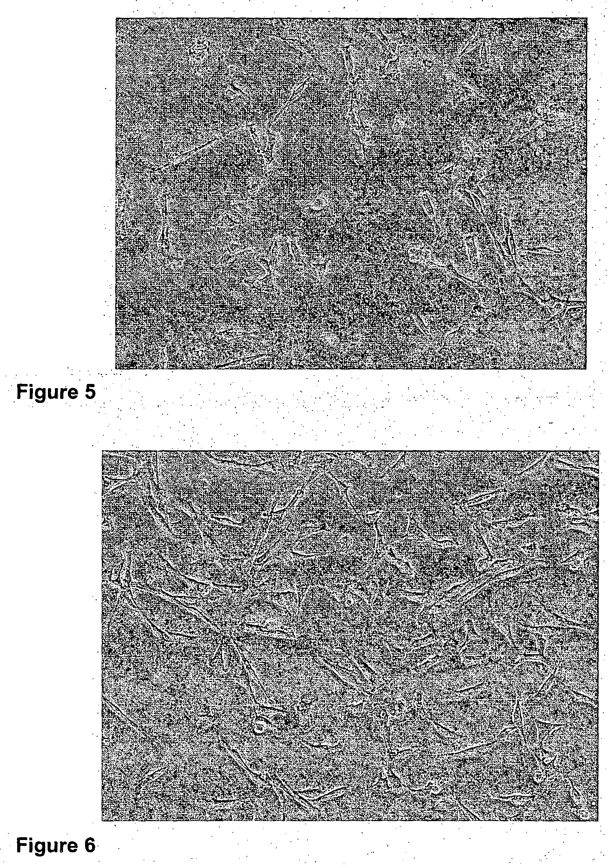

**Figure 5** shows a microscopic image of isolated cell samples (at day 4 of culture; at 100x magnification) after homogenization for 65 seconds. Figure 5 shows the microscopic image of cellular morphology of cells recovered using one method of the present disclosure.
**Figure 6** shows a microscopic image of isolated cell samples (at day 4 of culture; at 100x magnification) after mincing for 5 minutes. Figure 6 shows the microscopic image of cellular morphology of cells recovered using one method of the present disclosure.

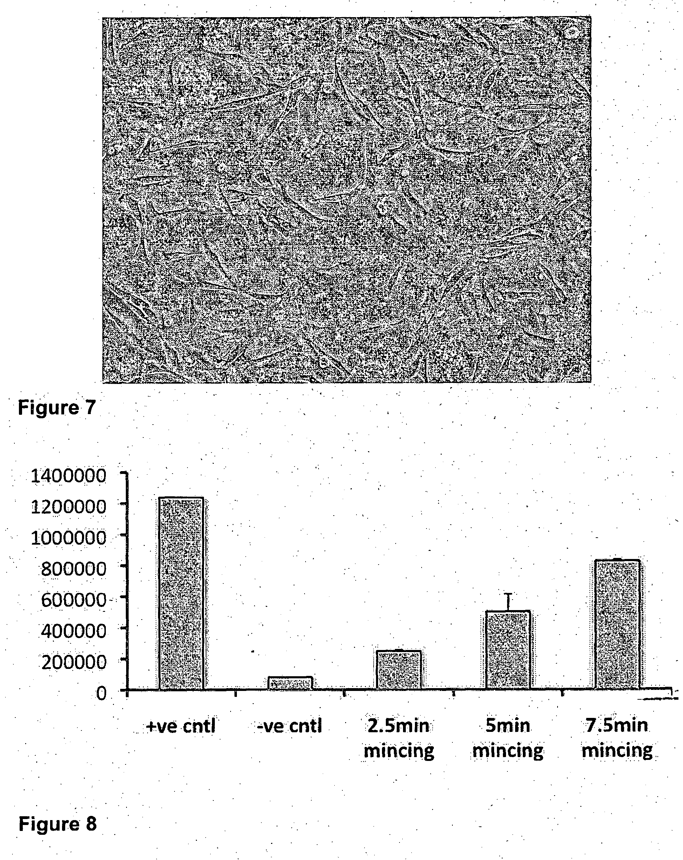

**Figure 7** shows a microscopic image of isolated cell samples (at day 4 of culture; at 100x magnification) after pre-treatment at 37°C for 1 hour followed by mincing for 5 mins. Figure 7 shows the microscopic image of cellular morphology of cells recovered using one method of the present disclosure.
**Figure 8** shows a bar graph depicting the number of live cell counts obtained after performing one of the methods of the present disclosure (i.e. mincing). In particular, the bar graph shows live cell counts per 5 ml of isolated fat tissue (at day 7 of culture). Mincing groups' measurements are the average of three samples. Data representative of at least 10 independent experiments. Positive control was obtained using method known in the art, which uses enzymatic digestion; negative control was mincing for 10 seconds.

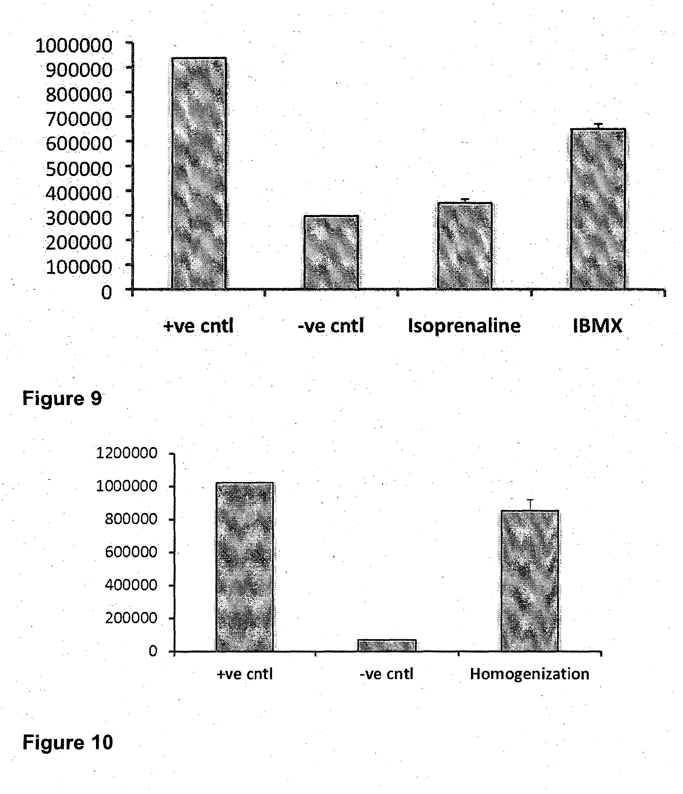

**Figure 9** shows a bar graph depicting the number of live cell counts obtained after performing one of the methods of the present disclosure in combination with lipolytic agents (isoprenaline and isobutylmethylxanthine- IBMX). In particular, the bar graph shows live cell counts per 5ml of isolated fat tissue (at day 7 of culture). Agent groups' measurements are the average of two samples. Data representative of at least five independent experiments. Positive control was obtained using method known in the art, which uses enzymatic digestion; negative control was mincing for 30 seconds.
**Figure 10** shows a bar graph depicting the number of live cell counts obtained after performing one of the methods of the present disclosure (i.e. mincing for 10 seconds followed by homogenization for 65 seconds). Homogenization groups' measurements are the average of three samples. Data representative of at least seven independent experiments. Positive control was obtained using method known in the art, which uses enzymatic digestion; negative control was mincing for 10 seconds.

### BRIEF DESCRIPTION OF THE TABLES

The invention will be better understood with reference to the detailed description when considered in conjunction with the non-limiting examples and the accompanying tables, in which:
**Table 1** shows the percentage yield of adipose-derived stem cells recovered using the methods of the present disclosure.
**Table 2** shows the percentage yield of adipose-derived stem cells recovered using the methods of the present disclosure, wherein the method further comprises the use of non-enzymatic agents as described herein.
**Table 3** shows the percentage yield of adipose-derived stem cells recovered using the methods of the present disclosure, wherein the method further comprises a combination of other methods of isolation.

### DETAILED DESCRIPTION OF THE PRESENT INVENTION

In view of the immense possibilities adipose-derived stem cells, which are derived from stromal vascular fraction, can provide in medicine, there is a need to have a method for recovering stromal vascular fraction. Accordingly, in one aspect, there is provided an alternative method for recovering stromal vascular fraction from a sample. As used herein, the phrase "stromal vascular fraction", "cells of stromal vascular fraction" or "stromal vascular fraction cells" refers to a cell fraction derived from vascular (or relating to vessels carrying blood) component of adipose tissue that comprises different cell types including, but not limited to, adipose-derived stem cells (e.g. mesenchymal stem cells), mesenchymal cells, hematopoietic cells, hematopoietic stem / progenitor cells, chondrocytes, osteoblasts, osteoclasts, endothelial precursor or progenitor cells, endothelial cells, smooth muscle cells, pericytes, CD34+ cells (typically found in umbilical cord), CD29+ cells, CD166+ cells, Thy-1+ or CD90+ stem cells, CD44+ cells, immune cells such as monocytes, leukocytes, lymphocytes, B and T cells, NK cells, macrophages, dendritic cells, neutrophil leukocytes, neutrophils, eosinophils, basophils, granulocytes, erythrocytes, megakaryocytes, platelets, and the like including immune and other cells that express one or more of the following markers: CD3, CD14 (macrophage marker), CD19, CD20 (B cell marker), CD29 (integrin unit), CD31 (endothelial, platelet, macrophage, dendritic cell, granulocyte, T/NK cells, lymphocytes, megakaryocytes, osteoclasts, neutrophils, *et al.*)*,* CD44 (Hyaluronic acid receptor), CD45 (B and T cell marker), CD56, CD73 (lymphocyte differentiation marker) and CD105. Also, stromal vascular fraction may also include cells expressing any of the markers or any combination thereof disclosed in the present disclosure. As used herein and in the art, the phrases "precursor cell," "progenitor cell," and "stem cell" may be used interchangeably and refer to either a pluripotent or lineage-uncommitted progenitor cell, which is potentially capable of an unlimited number of mitotic divisions to either renew itself or to produce progeny cells which will differentiate into the desired cell type. In contrast to pluripotent stem cells, lineage-committed progenitor cells are generally considered to be incapable of giving rise to numerous cell types that phenotypically differ from each other.

As used herein, the term "recovering", "isolating", or "harvesting" refers to the process of extracting the desired fraction or components of a tissue from its surrounding matrix that is commonly associated with the fraction or components of a tissue. In one example, the term "recovering", "isolating", or "harvesting" refers to the act of extracting the stromal vascular fraction from adipose tissue. In another example, the term "recovering", "isolating", or "harvesting" refers to the act of extracting the adipose-derived stem cell from the stromal vascular fraction as obtained from the method of the present disclosure.

As known method for recovering stromal vascular fraction typically involves dissociation enzymes such as collagenase, the present disclosure provides a non-enzymatic method for recovering stromal vascular fraction from a sample. That is, the method as described herein may not comprise enzyme digestion, which is in contrast to the method known in the art that comprises the step of adding enzymes to the sample prior to or at the time of performing the method of recovering the stromal vascular fraction. In other words, the method as described herein may not comprise exogenous enzyme digestion. As used herein the term "enzymes" refers to biological molecules that are capable of digesting extracellular matrix that binds cells together to form tissue. In the present disclosure, the enzymes may be dissociating enzymes that facilitate the digestion or dissociation of cell-to-cell contact such as collagenase, trypsin, and the like that can facilitate digestion of a tissue to single cell suspension. The exclusion of enzymes in the method of the present disclosure is to ensure recovery of stromal vascular fraction and the eventual adipose-derived stem cells that are of clinical grade and has minimal contamination risks. As used herein, the term "digestion", "digesting", or any other grammatical permutations that can be used interchangeably in the art, refers to the process of breaking down extracellular matrix that binds cells together to form tissue.

The method of the present disclosure comprises providing a mechanical force capable of breaking the sample into a single cell suspension whilst maintaining intact stromal vascular fraction cells cellular structures, wherein the mechanical force is mincing and/or homogenization. Thus, in one example, the method provides for a mechanical force that is not in combination with enzyme digestion. This reduces the need for repeated washing of sample or isolated stromal vascular fraction and reduces contamination risks. "Contamination", as used herein, refers to the presence of unwanted material such as exogenous enzymes, microorganism such as bacteria or fragment of the bacteria that may be detrimental to the health of the isolated cells and/or the health of the subject receiving the isolated cells or fraction. As known in the art, minimally manipulated cellular and tissue-based products is preferable in the art, which is in contrast to methods which involves enzyme digestions, which is known to be highlighted by the US Food and Drug Administration to be considered more than minimally manipulated and may pose public safety concerns. The mechanical forces of the present disclosure are provided at sufficient mechanical or shear force that is capable of causing the dissociation of cell-to-cell contact, thus leading to the desired single cell suspension, whilst maintaining intact stromal vascular fraction cells cellular structures. Thus, whilst the mechanical force provided in the present invention may disrupt fat cells, the mechanical force would not cause cells of stromal vascular fraction to burst or be destroyed. The mechanical force provided in the present invention would not cause cells of stromal vascular fraction to become unhealthy, thus enabling them to proliferate upon culturing in suitable conditions known in the art. In contrast, methods known in the art, in particular those that utilize enzyme digestions, are known to alter cellular behavior and gene expression of stromal vascular fractions cells. Accordingly, in one example, the mechanical forces excludes excessive forces that may cause cellular damage or cell rupture, for example, standard homogenizers and/or high power homogenization. As used herein, the phrase "single cell suspension", or equivalent expressions, refers to a mixture of a fluid and a cell, or more typically a plurality of cells, that are substantially or completely separated from each other (i.e. not aggregated or form clumps), which in the present disclosure may be prepared by any available mechanical, biological or chemical means.

As such, the method of the present disclosure does not comprise an internal mechanical force capable of breaking the sample into a single cell suspension. As used herein, the term "internal" refers to inside or within the sample and directly connected or in contact with the sample. An example of internal mechanical forces may include beads to be provided within or mixed with the sample. As illustrated in Table 3, the use of glass beads in combination with the method of the present disclosure resulted in a yield of less than 60% adipose-derived stem cells at 7 days of growth in culture as compared to a known enzymatic method of recovering stromal vascular fraction. Another example of internal mechanical force may include probe sonicator wherein the probe is in direct (fluid) contact with the sample. As illustrated in Table 1, the use of probe sonicator in the method of the present disclosure negatively affected cellular health and growth, and resulted in a yield of less than 40% adipose-derived stem cells at 7 days of growth in culture as compared to a known enzymatic method of recovering stromal vascular fraction. Accordingly, in one example, the method of the present disclosure may not be performed with a probe sonicator.

Without wishing to be bound by theory, it is believed that the method of the present disclosure may be used for recovering stromal vascular fraction from a sample that is known to have adipose-derived stem cells, such as, but is not limited to, adipose tissue. As used herein, the phrase "adipose tissue," refers broadly to any fat tissue. The adipose tissue may comprise mature adipocytes and other heterogenous cell populations, which upon isolation are termed stromal vascular fraction. In the present disclosure adipose tissue may be brown adipose tissue, white adipose tissue, mesenchymal or stromal. As used herein, "brown adipose tissue" and "white adipose tissue" refer to the types of adipose tissue that can be found in a mammal. Brown adipose tissue would typically be found in abundance in newborns or in hibernating mammals and contain more mitochondria than white adipose tissue. In one example, the adipose tissue may be subcutaneous white adipose tissue. The adipose tissue may be from any organism having fat tissue. In one example, the sample to be used in the present disclosure may be obtained from an animal, mammal, human, including, without limitation, animals such as cattle, pigs, horses, dogs, cats, rodents such as mice, rats, rabbits or guinea pigs, sheep, goats, dolphins and the like. In one example, the sample may be obtained from a mammal, wherein the mammal may be a human. In one example, the sample may be obtained in the form of lipoaspirate or resected fat that is derived from liposuction surgery or other surgeries.

To ensure maximal yield of stromal vascular fraction and cell viability, immediate use of the method for recovering of stromal vascular fraction of the present disclosure on the sample may be performed. However, if immediate recovery of stromal vascular fraction from the sample may not be performed immediately, the method of the present disclosure may be performed on the sample within about 1 hour to 72 hours. That is, the method of the present disclosure may be performed at about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, or 72 hours of the sample being obtained from a subject. When the sample is not processed immediately, the sample may be stored at about 4°C to reduce endogenous enzyme digestion or cell death.

As used herein, the term "endogenous enzymes" refers to enzymes that are intrinsically present in the sample, which is in contrast to the term "exogenous enzymes", which as used herein, refers to enzymes that are added on to the sample by the person performing a method of recovering stromal vascular fraction known in the art. Thus, in one example, the method of recovering stromal vascular fraction of the present disclosure does not comprise exogenous enzyme, i.e. added enzymes.

In one example, before the method of the present disclosure is performed on the sample, the sample may be stored in biologically acceptable liquid, such as but is not limited to phosphate buffered saline, Hank's balanced salt solution, normal saline, tumescent fluid and the like.

In one example, the method of the present disclosure may be performed at a range of about 0°C to about 42°C. Thus, the method of the present disclosure may be performed at about 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C or 42°C. In one example, the method of the present disclosure may be performed at about 25°C to about 42°C. In one example, to increase efficiency, the method of the present disclosure may be performed at 37°C, which can loosen collagen structures and enhance lipolytic activities of mature adipocytes.

To increase cell isolation efficiency, the method of the present disclosure may be performed with agents capable of enhancing lipolysis of fat cells. In one example, the method may further comprise a step of incubating the sample with agents capable of enhancing lipolysis of fat cells. In one example, the step of incubating the sample may be performed at a temperature ranging from about 25°C to about 42°C. That is, the step of incubating the sample may be performed at about 25°C, 26°C, 27°C, 28°C, 29°C, 30°C, 31°C, 32°C, 33°C, 34°C, 35°C, 36°C, 37°C, 38°C, 39°C, 40°C, 41°C or 42°C. In one example, the step of incubating the sample with the agent may be performed for about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 minutes. In one example, the step of incubating the sample with the agent may be performed for about 5 minutes, 15 minutes, 30 minutes, 45 minutes, 1, 1.5, or 2 hours.

These non-enzymatic agents capable of enhancing lipolysis of fat cells may include, but are not limited to, epinephrine, isobutylmethylxanthine, isoprenaline and the like. In one example, these agents may be used in combination with each other. Table 2 shows addition of these agents to the method of the present disclosure improves final field of adipose-derived stem cells. In one example, the epinephrine may be provided at a concentration of about 1 to 20 mg/L epinephrine. That is, the epinephrine may be provided at about 1, 2, 3, 4, 5, 6, 7, 8, 9, 10, 11, 12, 13, 14, 15, 16, 17, 18, 19, or 20 mg/L epinephrine. In another example, the isobutylmethylxanthine (IBMX) may be provided at a concentration of about 0.01 to 0.2 mM IBMX. That is, the IBMX may be provided at about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, or 0.20 mM IBMX. In another example, the isoprenaline may be provided at a concentration of about 0.01 to 0.20 mM isoprenaline. That is, the isoprenaline may be provided at about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, or 0.20 mM isoprenaline.

In another example, the method of the present disclosure may also be performed in a medium comprising phosphate buffered saline (PBS), Hank's balanced salt solution (HBSS) or erythrocyte lysis buffer.

To obtain the stromal vascular fraction, the method of the present disclosure may further comprise the step of isolating the stromal vascular fraction from the single cell suspension. The step of isolating the stromal vascular fraction of the present disclosure may be performed by density gradient, flow cytometry, membrane filtration, membrane adsorption, or centrifugation. When centrifugation is used as the step of isolating the stromal vascular fraction from the single cell suspension, it must be performed at optimal centrifugal force as excessive centrifugation may also lead to cellular damage. In one example, the centrifugal force may be performed at about 100 g, about 150 g, about 200 g, about 250 g, about 300 g, about 350 g, about 400 g, about 450 g, about 500 g, about 550 g, about 600 g, about 650 g, about 700 g, about 750 g, about 800 g, about 850 g, about 900 g, about 950 g, about 1000 g, about 1050 g, about 1100 g, about 1150 g, about 1200 g, about 1250 g, about 1300 g, about 1350 g, about 1400 g, about 1450 g or about 1500 g. The centrifugal force may be performed at a range of about 100 g, about 150 g, about 200 g, about 250 g, about 300 g, about 350 g, about 400 g, about 450 g, about 500 g, about 550 g, about 600 g, about 650 g, about 700 g, about 750 g, about 800 g, about 850 g, about 900 g, about 950 g, about 1000 g, about 1050 g, about 1100 g, about 1150 g, about 1200 g, about 1250 g, about 1300 g, about 1350 g, about 1400 g, about 1450 g to 100 g, about 150 g, about 200 g, about 250 g, about 300 g, about 350 g, about 400 g, about 450 g, about 500 g, about 550 g, about 600 g, about 650 g, about 700 g, about 750 g, about 800 g, about 850 g, about 900 g, about 950 g, about 1000 g, about 1050 g, about 1100 g, about 1150 g, about 1200 g, about 1250 g, about 1300 g, about 1350 g, about 1400 g, about 1450 g or about 1500 g, or any integers there between.

Upon recovery of stromal vascular fraction from sample, the method of the present disclosure may further comprise the step of isolating adipose-derived stem cells from the stromal vascular fraction. As used herein, the term "adipose-derived stem cell (ADSC or ASC)," refers to stromal or mesenchymal stem cells that from parts of the vascular elements found in adipose tissue (i.e. stromal vascular fraction). In one example, the adipose-derived stem cells may constitute about 0.5% to about 25% of the recovered or isolated stromal vascular fraction. Thus, in one example, the adipose-derived stem cells may constitute about 0.5%, about 1%, about 1.5%, about 2%, about 2.5%, about 3%, about 3.5%, about 4%, about 4.5%, about 5%, about 5.5%, about 6%, about 7%, about 7.5%, about 8%, about 8.5%, about 9%, about 9.5%, about 10%, about 10.5%, about 11%, about 11.5%, about 12%, about 12.5%, about 13%, about 13.5%, about 14%, about 14.5%, about 15%, about 15.5%, about 16%, about 17%, about 17.5%, about 18%, about 18.5%, about 19%, about 19.5%, about 20%, about 20.5%, about 21%, about 21.5%, about 22%, about 22.5%, about 23%, about 23.5%, about 24%, about 24.5%, about 25%, or any amount there between of the recovered or isolated stromal vascular fraction. In one example, the adipose-derived stem cell may be differentiated into various cell types under conditions known in the art. For example, the adipose-derived stem cell as obtained from the method of the present disclosure may be trans-differentiated into non-mesodermal cell types, such as, but is not limited to, neuronal cells, hepatocytes and endothelial cells; the adipose-derived stem cell as obtained from the method of the present disclosure may be differentiated into mesodermal lineages cell types, such as, but is not limited to adipocyte, fibroblasts, myocytes, cardiomyocytes, osteoblasts and chondrocytes. It would be understood in the art that the process of differentiation of adipose-derived stem cells may be performed under standard culturing procedures or other methods known in the art. The adipose-derived stem cells may be isolated from the stromal vascular fraction using techniques known in the art, for example, the cells can be isolated from a mixture of cells using the cell surface markers known to be expressed by adipose-derived stem cells. In one example, the term "adipose-derived stem cell" includes cells which exhibit characteristics such as plastic adherent in standard culture conditions, express CD105, CD73 and CD90, but negative for the surface expression of CD45, CD34, CD14, CD79 and HLA-DR and able to differentiate into osteoblast, adipocytes and chondroblasts *in vitro.*

As used herein, the term "about", in the context of percentage of cells with respect with the total stromal vascular fraction recovered, may mean +/- 0.5% of the stated value, +/-0.4% of the stated value, +/- 0.3% of the stated value, +/- 0.2% of the stated value, +/- 0.1% of the stated value, or +/- 0.05% of the stated value.

In one example, the adipose-derived stem cells may be cultured before administered to a subject in need thereof. In one example, the cells as isolated or recovered from the method of the present disclosure may be cultured in tissue culture medium such as, but is not limited to, minimum essential media (MEM), Dulbecco's Modified Eagle Medium (DMEM), Roswell Park Memorial Institute (RPMI)-1640 and DMEM:F12. It is understood that the selection of medium would advantageously lead to a differential expression of genes in the cultured adipose-derived stem cells.

In another example, stromal vascular fraction cells and/or adipose-derived stem cells isolated from the stromal vascular fraction may be cultured in medium with human serum, such as allogenic AB serum (serum obtained from AB blood type donors / serum that does not contain anti-type A antibodies or anti-type B antibodies), thrombin-activated platelet-rich plasma and human platelet lysate. In another example, the stromal vascular fraction cells and/or adipose-derived stem cells may be cultured in serum-free medium. Addition of serum into the tissue culture medium containing the cells as isolated by the method of the present disclosure would advantageously lead to the improvement in total cellular yield.

The term "isolated" or "recovered" as used herein with respect to adipose-derived stem cells or stromal vascular fraction, refers to cells separated from constituents in which the cells are normally associated in nature. Thus, in one example, the present disclosure also provides isolated adipose-derived stem cells and/or isolated stromal vascular fraction.

In view of the usefulness of the adipose-derived stem cells, the adipose-derived stem cells may be used in a variety of methods of treating a subject in need of cell therapy. Accordingly, described herein is a method of treating a subject in need of cell therapy, wherein the method comprises administering the cells of the stromal fraction obtained from the method of the present disclosure to the subject in need thereof. In one example, the subject or patient, which term may be used interchangeably, may be an animal, mammal, human, including, without limitation, animals such as cattle, pigs, horses, dogs, cats, rodents such as rabbits, mouse, rats, or guinea pigs, dolphins, sheep, goats, and the like. In one example, the patient may be a human. In one example, there is provided the use of the cells of the stromal fraction obtained from the method of the present disclosure in the manufacture of a medicament for treating a subject in need of cell therapy.

Also described herein is a method of treating a subject in need of cell therapy, wherein the method comprises the adipose-derived stem cells obtained from the method of the present disclosure to the subject in need of cell therapy. In one example, the subject or patient, which term may be used interchangeably, may be an animal, mammal, human, including, without limitation, animals such as cattle, pigs, horses, dogs, cats, rodents such as rabbits, mouse, rats, or guinea pigs, dolphins, sheep, goats, and the like. In one example, the patient may be a human. In one example, there is provided the use of the adipose-derived stem cells obtained from the method of the present disclosure in the manufacture of a medicament for treating a subject in need of cell therapy.

In one example, the cell therapy may be used in subjects for numerous clinical applications as described in Lim, M.H., *et al.* 2014, the content of which is incorporated herewith by reference. For example, the cell therapy using the cells of stromal vascular fraction obtained from the method of the present disclosure and /or the adipose-derived stem cells may be used for subjects who may require any one of the following: tissue grafts, bone and cartilage repair, repair from ischemic diseases, repair from peripheral vascular diseases, soft tissue augmentation and reconstructive surgery, wound healing, repair from or treatment of immune diseases, repair from or treatment of diabetes mellitus and the like. For example, hematopoietic stem or progenitor cells may be useful for treating blood diseases, and endothelial cells may be useful for vascularization and formation of functional tissues in tissue grafting. As used herein, the term "graft" refers to a cell, tissue or organ that is implanted into an individual, typically to replace, correct or otherwise overcome a defect. A graft may further comprise a scaffold. The tissue or organ may consist of cells that originate from the same individual; this graft is referred to herein by the following interchangeable terms: "autograft", "autologous transplant", "autologous implant" and "autologous graft". A graft comprising cells from a genetically different individual of the same species is referred to herein by the following interchangeable terms: "allograft", "allogeneic transplant", "allogeneic implant" and "allogeneic graft". "Allogenic" refers broadly to any material derived from a different mammal of the same species, for example another human who is not the recipient of the graft. A graft from an individual to his identical twin is referred to herein as an "isograft", a "syngeneic transplant", a "syngeneic implant" or a "syngeneic graft". A "xenograft", "xenogeneic transplant" or "xenogeneic implant" refers to a graft from one individual to another of a different species.

In another aspect, there is provided a method of recovering stromal vascular fraction from a sample comprising subjecting the sample to mincing. As used herein, the term "mincing", "cutting" or any other synonyms referring to the same act, refers to the act of cutting up the sample into very small pieces. The process "mincing" or "cutting" may be performed manually by the technician performing the method or operated by a device or apparatus for mincing and/or cutting. In one example, the mincing or cutting process may be applied for about 10 seconds to 20 minutes. That is, the mincing process may be applied for about 10 seconds, 15 seconds, 20 seconds, 25 seconds, 30 seconds, 35 seconds, 40 seconds, 45 seconds, 50 seconds, 55 seconds, 1 minutes, 2 minutes, 3 minutes, 4 minutes, 5 minutes, 6 minutes, 7 minutes, 8 minutes, 9 minutes, 10 minutes, 11 minutes, 12 minutes, 13 minutes, 14 minutes, 15 minutes, 16 minutes, 17 minutes, 18 minutes, 19 minutes, 20 minutes, or any integers there between.

In another aspect, there is provided a method of recovering stromal vascular fraction from a sample comprising subjecting the sample to homogenization. In one example, the homogenizer may be a low-powered homogenizer such as Labquip (IKA T18 Basic Ultra-Turrax) and the like. In one example, the homogenizer may provide circumferential speeds of about 1,000 to about 10,000 rpm. Thus, in one example, the homogenizer may be carried out at about 1000 rpm, about 1050 rpm, about 2000 rpm, about 2500 rpm, about 3000 rpm, about 3500 rpm, about 4000 rpm, about 4500 rpm, about 5000 rpm, about 5500 rpm, about 6000 rpm, about 6500 rpm, about 7000 rpm, about 7500 rpm, about 8000 rpm, about 8500 rpm, about 9000 rpm, about 9500 rpm, about 10,000 rpm or any integers there between. In one example, the homogenization process or residence times in the homogenization vessel may be in the range of about 5 seconds to 20 minutes. That is, the homogenization process may be applied for about 5 seconds, 10 seconds, 15 seconds, 20 seconds, 25 seconds, 30 seconds, 35 seconds, 40 seconds, 45 seconds, 50 seconds, 55 seconds, 1 minutes, 2 minutes, 3 minutes, 4 minutes, 5 minutes, 6 minutes, 7 minutes, 8 minutes, 9 minutes, 10 minutes, 11 minutes, 12 minutes, 13 minutes, 14 minutes, 15 minutes, 16 minutes, 17 minutes, 18 minutes, 19 minutes, 20 minutes, or any integers there between. In one example, the method for recovering stromal vascular fraction of the present aspect may further comprise the step of isolating the stromal vascular fraction that may be performed by density gradient, flow cytometry, membrane filtration /adsorption or centrifugation. In another example, the method of the present aspect may further comprise resuspending the stromal vascular fraction in suitable media or buffer as described herein.
In one example, the method of the present aspect may further comprise a step of mincing the sample prior to subjecting the sample to homogenization. In one example, the mincing or cutting process may be performed for about 1 minute, 2 minutes, 3 minutes, 4 minutes, 5 minutes, 6 minutes, 7 minutes, 8 minutes, 9 minutes, 10 minutes, 11 minutes, 12 minutes, 13 minutes, 14 minutes, 15 minutes, 16 minutes, 17 minutes, 18 minutes, 19 minutes, 20 minutes or any integers there between.

In another aspect, there is provided a method of cryopreserving stromal vascular fraction and/or adipose-derived stem cells. The method may comprise recovering stromal vascular fraction and/or adipose-derived stem cells from a sample, washing the isolated cells of stromal vascular fraction and/or adipose-derived stem cells with buffer and/or media, freezing the isolated cells of stromal vascular fraction and/or adipose-derived stem cells in suitable media. In one example, suitable media for cryopreserving stromal vascular fraction and/or adipose-derived stem cells may include growth media containing 10% serum (such as fetal bovine serum or human AB serum) and 10 % DMSO (dimethyl sulfoxide).

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

### EXPERIMENTAL SECTION

### EXAMPLE 1 - PREPARATION OF SAMPLE AFTER HARVEST

### Method

Fat tissue (adipose tissue) obtained as lipoaspirate or resected via surgeries are to be obtained prior conducting any of the methods as described herein. To ensure optimum cell viability, immediate isolation of stromal vascular fraction on the day the sample is obtained is recommended. However, the methods as described herein may be performed optimally within 24 hours of sample being obtained. If the methods of the present disclosure cannot be performed immediately upon harvest of sample or within 72 hours of the sample being obtained, the sample was stored at 4°C. In case of enzymatic digestion, the sample is brought to room temperature before proceeding to the isolation procedure.

Upon obtaining the sample as lipoaspirate, to remove debris, the fat tissue was washed with equal amount of buffer (such as phosphate buffered saline (PBS) or Hank's balanced salt solution (HBSS)). For example, for 50 cc or 50 gram volume of lipoaspirate, 50 ml of buffer was used. Washed sample was allowed to settle for a couple of minutes and fluid from the bottom was aspirated to leave fat layer in the top. Washing steps were repeated two times. Collected fat layer was divided into the different methods as described below.

### Materials

To increase efficiency of cell isolation, any of the methods as described herein were combined with other variables, such as altered operation temperature ranging from room temperature (25°C) to 42°C, addition of chemicals to enhance lipolysis of mature fat cells (e.g. 5mg/L epinephrine, 0.05 mM Isobutylmethylxanthine (IBMX), or 0.05mM isoprenaline) and use of different buffers during mechanical treatment (e.g. erythrocyte lysis buffer instead of HBSS).

### EXAMPLE 2 - TRADITIONAL ENZYMATIC METHOD

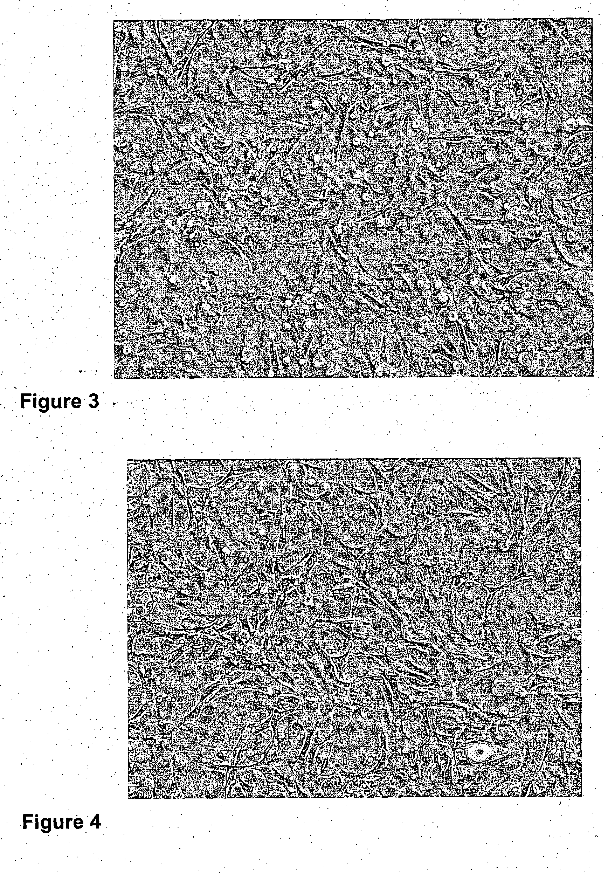

Known enzymatic method for recovering stromal vascular fraction has been described previously (WO2011/032025, Sugii *et al.,* 2010, and Sugii *et al.,* 2011). Briefly, the known/traditional enzymatic method comprises the following steps:
i) Add an equal volume of collagenase solution (ml per gram or cc of fat pads) into one sterile vial and warm to 37°C. Typically, collagenase solution is composed of 2.5 µg/ml collagenase type I, 1% (wt/vol) Bovine Serum Albumin (BSA), 50 µg/ml D-glucose and 200 nM adenosine in buffer;
ii) Place washed fat tissue into collagenase solution;
iii) Finely mince the tissue with a sterilized scissor;
iv) Shake vials (∼100 rpm) in a 37°C water bath shaker for 30 - 60 min. Check digests every 10 min and stop the reaction when it is complete;
v) Transfer and filter the digested solution through a 250 µm nylon filter. Then filter through a 100 µm cell strainer and transfer the filtrate into 15 and 50 ml centrifuge tubes, depending on the volume. Centrifuge at 400g for 5 min at room temperature;
vi) Carefully aspirate the floating layer containing mature adipocytes and aqueous supernatant, leaving the pellet. This pellet is the stromal vascular fraction;
vii) Re-suspend the pellet with 10 ml buffer for washing. Centrifuge again at 400g for 5 min;
viii) Repeat step vii) three times;
ix) If the pellet appears red, rupture the red blood cells by adding 10 ml erythrocyte lysis buffer (154 mM NH₄Cl and 20 mM Tris, pH7.4). Pipette up and down to resuspend the pellet. The solution should become red. Leave for 5 min at room temperature and centrifuge at 400g for 5 min. Aspirate the supernatant;
x) For culturing, re-suspend the pellet in 10 ml culture medium (e.g. Dulbecco's Modified Eagle Medium (DMEM; Lifetechnology, USA), 15% heat inactivated fetal bovine serum (FBS; Lifetechnology, USA), 1x GlutaMAX™ (Lifetechnology, USA), 1x non-essential amino acids (NEAA; Lifetechnology, USA), 5 ng/ml basic fibroblast growth factor (FGF; Lifetechnology, USA), penicillin/streptomycin [100 U/ml / 100 µg/ml; Lifetechnology, USA] or commercial serum-free, xeno-free media).
xi) Plate into appropriate dishes and incubate culture plates in 37°C, 5% CO₂ incubator. Wash three times with buffer the next day, and change with fresh media. Observe under microscope to check growth and morphology (typical microscopic photo Figures 2 and 3);
xii) For immediate storage, count the cell number with hemocytometer or automated cell counter. Resuspend the pellet in freezing media (DMEM with 15% FBS and 10% dimethyl sulfoxide (DMSO), or commercial serum-free, xeno-free storage solution) at 1 x 10⁶ viable cells per ml. Store 1 x 10⁶ cells per vial.

### EXAMPLE 3 - SONICATION METHOD (WATER BATH SONICATORS)

A detailed example of the steps of the method of the present disclosure, when using the sonication method, in particular water bath sonicators, are as follows:
i) Place washed fat tissue into appropriate sized tubes;
ii) Fill cooled water to the indicated level to avoid overheating of the water bath sonicator;
iii) Sonicate fat tissue samples at the lowest level setting (for example, 40 kHz using Branson's 3510 model) for 5 - 10 min. As would be apparent to the skilled addressee, different sonicators would give very different results. Hence, optimization of sonication time and intensity would need to be conducted;
iv) Centrifuge samples at 400g for 5 min to pellet dissociated stromal vascular fraction cells. Transfer supernatant to fresh tubes, and use this for the step vi) below if necessary;
v) Re-suspend pellet in buffer (PBS or HBSS; 1 ml per 100 cc original tissue). Count cell number with hemocytometer or automated cell counter;
vi) If cell number is far less than 1 x 10⁶ cells per 100 cc original tissue, repeat the steps iii) to v). If still low cell number recovery, increase the strength to the next level if available or repeat the steps iii) to v). With this method, up to 30% of cell yield as compared to enzyme-based method in Example 1 was achieved;
vii) For culturing, add 10 ml culture medium (e.g. DMEM, 15% heat-inactivated FBS, 1x GlutaMAX™, 1x NEAA, 5 ng/ml basic FGF, penicillin/streptomycin [100 U/ml / 100 µg/ml] or commercial serum-free, xeno-free media) to the cells;
viii) Plate into appropriate dishes and incubate culture plates in 37°C, 5% CO₂ incubator. Wash three times with buffer the next day, and change with fresh media. Observe under microscope to check growth;
ix) For immediate storage, resuspend the pellet in freezing media (DMEM with 15% FBS and 10% DMSO, or commercial serum-free, xeno-free storage solution) at 1 x 10⁶ viable cells per ml. Store 1 x 10⁶ cells per vial.

### EXAMPLE 4 - SONICATION METHOD (PROBE SONICATORS)

A detailed example of the steps of the method of the present disclosure, when using the sonication method, in particular probe sonicators, are as follows:
i) Place washed fat tissue into appropriate sized tubes;
ii) Sterilize the probe of the sonicator or homogenizer;
iii) For the initial experiment, sonicate fat tissue samples for 0.5-second stroke followed by 0.5-second interval at the lowest level setting for 1 min. Combinations of various parameters should be tested; energy range from 1.5 to 3 MHz, from 5 to 30 strokes with from 0.5 to 1.5 second pulses. As would be apparent to the skilled addressee, different sonicators would give very different results. Hence, optimization of sonication time and intensity would need to be conducted;
iv) Centrifuge samples at 400g for 5 min to pellet dissociated stromal vascular fraction cells. Transfer supernatant to fresh tubes, and use this for the step vi) below if necessary.
v) Re-suspend pellet in buffer (PBS or HBSS; 1 ml per 100 cc original tissue). Count cell number with hemocytometer or automated cell counter;
vi) If cell number is less than 1 x 10⁶ cells per 100 cc original tissue, repeat steps iii) to v). If cell recovered is still low, adjust the level and length as described in the step iii). With this method, up to 40% of cell yield compared to enzyme-based method in Example 1 was achieved;
vii) For culturing, add 10 ml culture medium (e.g. DMEM, 15% FBS, 1x GlutaMAX™, 1x NEAA, 5 ng/ml basic FGF, penicillin/streptomycin [100 U/ml / 100 µg/ml] or commercial serum-free, xeno-free media) to the cells;
viii) Plate into appropriate dishes and incubate culture plates in 37°C, 5% CO₂ incubator. Wash three times with buffer the next day, and change with fresh media. Observe under microscope to check growth;
ix) For immediate storage, resuspend the pellet in freezing media (DMEM with 15% FBS and 10% DMSO, or commercial serum-free, xeno-free storage solution) at 1 x 10⁶ viable cells per ml. Store 1 x 10⁶ cells per vial.

### EXAMPLE 5 - OTHER MECHANICAL METHODS

A detailed example of the steps of the method of the present disclosure, when using other mechanical methods, such as scissors and homogenizers, are as follows:
i) Place washed fat tissue into appropriate tubes. Add equal volume of HBSS solution. Usually 10 ml of fat is used for each variable for replicate treatment/plating. Resected fat is favorable for consistency of cell yield, but manually harvested lipoaspirates are acceptable though additional optimization work described below is recommended;
ii) Sterilize the mechanical component (e.g. scissors, homogenizer probes);
iii) Mount a pair of sterilized homogenizers or scissors onto the containers holding fat tissue. Carefully place each 50 ml tube of fat into the holder on the platform and adjust the height of the tube so that the homogenizers or scissors nearly touch the bottom of the tube. Switch on and adjust speed of rotation and start timer to start homogenization or cutting;
iv) Mince the tissue until fine; typically 5-12 minutes for treatment, depending on softness of harvested fat tissue. When homogenizer is to be used, mildly homogenize fat tissue samples at the lowest level setting for 1 min. As would be apparent to the skilled person in the art, different homogenizers would give very different results. Many standard homogenizers in market utilize power levels that are too strong and often not suitable for the present method. Optimization for different type of fat tissues must be conducted to obtain optimized parameters and lengths of homogenizing time;
v) When homogenization or mincing is done, switch off rotation motor and carefully remove centrifuge tube;
vi) Centrifuge samples at 450g (about 1800 RPM) for 5 min to pellet dissociated stromal vascular fraction cells. If large chunks of fat are present, increase centrifugation to 1200g (about 2950 RPM). Discard the top layer of floating mature adipocytes;
vii) Transfer remaining solution through a 100 µm cell strainer. Re-suspend the red pellet with 4 ml of HBSS and pour it through the strainer. Flush the cell strainer with HBSS;
viii) Centrifuge at 400g for 5 minutes at room temperature;
ix) Aspirate the floating layer containing mature adipocytes and aqueous supernatants, leaving pellet. This pellet is the stromal vascular fraction;
x) Re-suspend pellet in 2 ml of ACK Lysis Buffer. Incubate for 5 minutes at room temperature;
xi) Centrifuge at 450g for 5 minutes at room temperature;
xii) Re-suspend pellet in buffer (PBS or HBSS; 1 ml per 100 cc original tissue). Sample out 10 µl and count cell number in 1:1 mixture of trypan blue by hemocytometer or automated cell counter. With these methods, we have achieved more than 70% of cell recovery compared to the enzyme-based method in A).
xiii) If cell number is less than 1 x 10⁷ cells per 100 cc original tissue, repeat the Step iv) to xii). If cell number recovered is still low, adjust the level and length as described in the step iv);
xiv) For culturing, add 10 ml culture medium per 1 x 10⁶ cells (e.g. DMEM, 15% heat-inactivated FBS, 1x GlutaMAX™, 1x NEAA, 5 ng/ml basic FGF, penicillin/streptomycin [100 U/ml / 100 µg/ml] or commercial serum-free, xeno-free media) to the cells;
xv) Plate into appropriate dishes and incubate culture plates in 37°C, 5% CO₂ incubator. Wash three times with buffer the next day, and change with fresh media. Observe under microscope to check growth;
xvi) For immediate storage, re-suspend the pellet in freezing media (DMEM with 15% FBS and 10% DMSO, or commercial serum-free, xeno-free storage solution) at 1 x 10⁶ viable cells per ml. Store 1 x 10⁶ cells per vial.

### EXAMPLE 6 - ANALYSIS OF ISOLATED CELLS

The isolated stromal vascular fraction cells in the methods described above were analyzed for their qualities and yield of adipose-derived stem cells. The analyses include cell counting and viability with a hemocytometer, culturing to enrich adipose-derived stem cells, limiting dilution and colony formation assay, cell surface markers, and multipotent differentiation assays. These procedures were performed according to the published protocols (Sugii, S., *et al.* 2011; Katz, A.J., *et al.* 2005; Ong, W.K., *et al.,* 2014; Yang, Z *et al.,* 2011; and Zuk, P.A., *et al.,* 2001). Freshly isolated stromal vascular fraction cells are cultured in the growth medium in the media described above. Differentiation assays include adipogenesis, chondrogenesis and osteogenesis. For example, cells are induced by culturing in adipogenic induction medium (growth medium + 1.5 µg/ml insulin + 1.0 µM dexamethasone + 0.5 mM isobutylmethylxantine + 0.2 mM indomethacin) followed by adipogenic maintenance medium (growth medium + 1.5 µg/ml insulin). Osteogenesis and chondrogenesis were performed with StemPro kits available from GIBCO / LifeTech. Degrees of adipogenesis were assessed by staining with Oil Red O. Successful stromal vascular fraction isolation would generate at least 1 x 10⁵ healthy adipose-derived stem cell population per 1 cc or gram tissue, which possess colony formation capacity, >95% express cell surface markers, CD73, CD90 and CD105 and are capable of differentiating into adipocytes, chondrocytes and osteoblasts.

### EXAMPLE 7 - RESULTS OF ISOLATION ACCORDING TO THE METHODS OF THE PRESENT DISCLOSURE

**Table 1 shows the percentage yield of adipose-derived stem cells recovered using the methods of the present disclosure.**

| **Treatment** | **Protocol** | **Yield*** |
|---|---|---|
| Mincing 1 | Mince with scissors for 5-10 mins | >70 |
| Mincing 2 | Rotating cutting blades for 2-5 mins | >60 |
| Heat + Mincing | Mince 2.5 mins and incubate at 40°C for 1.5 hours | >75 |
| Homogenizer 1 | Large tissue homogenizer at (lowest setting) for 1-2 mins | >70 |
| Homogenizer 2 | Small tissue homogenizer (lowest setting) for 5-25 mins | <30 |
| Vortexing | Vortex for 0.5-1.5 mins | >60 |
| Probe Sonicator | Mince 2.5 mins and sonicate (lowest setting) with hand held unit for 5-25 mins | <40 |
| Water Bath Sonicator | Mince 2.5 mins and sonicate in water bath unit at 37-40°C for 1-30 mins | <30 |

| | | |
|---|---|---|
| *The yield shown is the percentage of ASCs observed after seven days of growth, compared to the collagenase-based method. Collagenase treated fat would normally show a nearly 100% yield. | | |

**Table 2** shows the percentage yield of adipose-derived stem cells recovered using the methods of the present disclosure, wherein the method further comprises the use of non-enzymatic agents of as described herein.

| **Treatment** | **Protocol** | **Yield** |
|---|---|---|
| Epinephrine | Mince 2.5 mins and incubate in 5mg/L epinephrine at 37°C for 1 hour. | >85 |
| Isobutylmethylxanthine (IBMX) | Mince 2.5 mins and incubate in 0.05mM IBMX at 37°C for 1 hour. | >80 |
| Isoprenaline | Mince 2.5 mins and incubate in 0.05mM isoprenaline at 37°C for 1 hour. | >70 |
| Sodium deoxycholate (SOC) | Mince 2.5 mins and incubate in 0.1-5% SOC at 37°C for 1 hour. | 0 |
| Triton-X | Mince 2.5 mins and incubate in 0.005-10mM Triton-X at 37°C for 1 hour. | 0 |

**Table 3 shows the percentage yield of adipose-derived stem cells recovered using the methods of the present disclosure, wherein the method further comprises a combination of other methods of isolation.**

| **Treatment** | **Protocol** | **Yield** |
|---|---|---|
| Mincing + erythrocyte lysis buffer (eLB) | Mince 5+ mins in eLB and incubate at 37°C for 15 mins. Centrifuge at 900xg for 15 mins | >65 |
| Heat + Glass Beads | Mince 5+ mins and vortex/hand shake; incubate at 40-42°C for 1 hour | <60 |
| Heat + pH (adjust by adding HCl or NaOH to HBSS buffer) | Mince 5+ mins and incubate at pH4-7.5 at 40-42°C for 1-3 hours | <30 |

### REFERENCES:

Sugii, S., et al., Feeder-dependent and feeder-independent iPS cell derivation from human and mouse adipose stem cells. Nat Protoc, 2011. 6(3): p. 346-58.
Sugii, S., et al., Human and mouse adipose-derived cells support feeder-independent induction of pluripotent stem cells. Proc Natl Acad Sci U S A, 2010. 107(8): p. 3558-63.
Katz, A.J., et al., Cell surface and transcriptional characterization of human adipose-derived adherent stromal (hADAS) cells. Stem cells, 2005. 23(3): p. 412-23.
Ong, W.K., et al., Identification of specific cell-surface markers of adipose-derived stem cells from subcutaneous and visceral fat depots. Stem Cell Reports, 2014. 2(2):p. 171-9.
Yang, Z., J.F. Schmitt, and E.H. Lee, Immunohistochemical analysis of human mesenchymal stem cells differentiating into chondrogenic, osteogenic, and adipogenic lineages. Methods in molecular biology, 2011. 698: p. 353-66.
Zuk, P.A., et al., Multilineage cells from human adipose tissue: implications for cell-based therapies. Tissue Eng, 2001. 7(2): p. 211-28.
Fraser, J.K., et al., Fat tissue: an underappreciated source of stem cells for biotechnology. Trends Biotechnol, 2006. 24(4): p. 150-4.
Sotiropoulou, P.A., et al., Characterization of the optimal culture conditions for clinical scale production of human mesenchymal stem cells. Stem cells, 2006.24(2):p.462-71.
Cawthorn, W.P., E.L. Scheller, and O.A. MacDougald, Adipose tissue stem cells: the great WAT hope. Trends Endocrinol Metab, 2012. 23(6): p. 270-7.
Gimble, J.M., A.J. Katz, and B.A. Bunnell, Adipose-derived stem cells for regenerative medicine. Circ Res, 2007. 100(9): p. 1249-60.
Ong, W.K. and S. Sugii, Adipose-derived stem cells: Fatty potentials for therapy. The international journal of biochemistry & cell biology, 2013. 45(6): p. 1083-1086.
Lim, M.H., W.K. Ong, and S. Sugii, The current landscape of adipose-derived stem cells in clinical applications. Expert reviews in molecular medicine, 2014. 16: p. e8.

## Claims

1. A method for recovering stromal vascular fraction from a sample, the method comprising providing a mechanical force capable of breaking the sample into a single cell suspension whilst maintaining intact stromal vascular fraction cells cellular structures, wherein the mechanical force is mincing and/or homogenization, wherein the method is a non-enzymatic digestion.

2. The method according to claim 1, wherein the sample is an adipose tissue.

3. The method according to claim 1, wherein the method further comprises the step of isolating the stromal vascular fraction from the single cell suspension.

4. The method according to claim 3, wherein the step of isolating the stromal vascular fraction is performed by density gradient, flow cytometry, membrane filtration, membrane adsorption or centrifugation.

5. The method according to claim 1, wherein the stromal vascular fraction comprises any one of the following cells: adipose-derived stem cells, mesenchymal cells, hematopoietic cells, hematopoietic stem / progenitor cells, chondrocytes, osteoblasts, osteoclasts, endothelial precursor or progenitor cells, endothelial cells, smooth muscle cells, pericytes, CD34+ cells, CD29+ cells, CD166+ cells, Thy-1+ or CD90+ stem cells, CD44+ cells and immune cells selected from the group consisting of monocytes, leukocytes, lymphocytes, B and T cells, NK cells, macrophages, dendritic cells, neutrophil leukocytes, neutrophils, eosinophils, basophils, granulocytes, erythrocytes, megakaryocytes, platelets.

6. The method according to claim 1, wherein the sample is obtained from a mammal.

7. The method according to claim 6, wherein the mammal is human.

8. The method according to claim 1, wherein the method is performed within 72 hours of harvest of the sample.

9. The method according to claim 1, wherein the method is performed at 0°C to 42°C.

10. The method according to claim 1, wherein the method is performed with agents which enhance lipolysis of fat cells, wherein the agent is selected from the group consisting of epinephrine, isobutylmethylxanthine and isoprenaline.

11. The method according to any one of claims 1 to 10, wherein the homogenization is carried out at 1000 rpm to 10,000 rpm.

12. The method according to any one of claims 1 to 11, wherein the mechanical force is applied for 5 seconds to 20 minutes.

13. The method according to claim 1, further comprising a step of isolating adipose-derived stem cells from the stromal vascular fraction obtained from the method of claim 1.

## Patentansprüche

1. Verfahren zum Gewinnen der stromalen vaskulären Fraktion aus einer Probe, wobei das Verfahren das Bereitstellen einer mechanischen Kraft umfasst, die die Probe in eine einzelne Zellsuspension brechen kann, während intakte Zellen der stromalen vaskulären Fraktion erhalten bleiben, wobei die mechanische Kraft Zerkleinern und/oder Homogenisieren ist, wobei das Verfahren eine nicht-enzymatische Spaltung ist.

2. Verfahren nach Anspruch 1, wobei die Probe ein Fettgewebe ist.

3. Verfahren nach Anspruch 1, wobei das Verfahren zudem den Schritt umfasst, bei dem die stromale vaskuläre Fraktion aus der Einzelzellsuspension isoliert wird.

4. Verfahren nach Anspruch 3, wobei der Schritt, bei dem die stromale vaskuläre Fraktion isoliert wird, durch Dichtegradient, Durchflusszytometrie, Membranfiltration, Membranadsorption oder Zentrifugation durchgeführt wird.

5. Verfahren nach Anspruch 1, wobei die stromale vaskuläre Fraktion irgendeine der folgenden Zellen umfasst: von Fettgewebe abgeleitete Stammzellen, mesenchymale Zellen, hämatopoetische Zellen, hämatopoetische Stamm-/Vorläuferzellen, Chondrozyten, Osteoblasten, Osteoklasten, endotheliale Vorstufen- oder Vorläuferzellen, Endothelzellen, glatte Muskelzellen, Perizyten, CD34+-Zellen, CD29+-Zellen, CD166+-Zellen, Thy-1+- oder CD90+-Stammzellen, CD44+-Zellen und Immunzellen ausgewählt aus der Gruppe bestehend aus Monozyten, Leukozyten, Lymphozyten, B- und T-Zellen , NK-Zellen, Makrophagen, Dendritenzellen, neutrophilen Leukozyten, Neutrophilen, Eosinophilen, Basophilen, Granulozyten, Erythrozyten, Megakaryozyten, Blutplättchen.

6. Verfahren nach Anspruch 1, wobei die Probe aus einem Säugetier erhalten wird.

7. Verfahren nach Anspruch 6, wobei das Säugetier ein Mensch ist.

8. Verfahren nach Anspruch 1, wobei das Verfahren innerhalb von 72 Stunden nach der Probenernte durchgeführt wird.

9. Verfahren nach Anspruch 1, wobei das Verfahren bei 0°C bis 42°C durchgeführt wird.

10. Verfahren nach Anspruch 1, wobei das Verfahren mit Mitteln durchgeführt wird, die die Lipolyse von Fettzellen verstärken, wobei das Mittel ausgewählt ist aus der Gruppe, bestehend aus Epinephrin, Isobutylmethylxanthin und Isoprenalin.

11. Verfahren nach einem der Ansprüche 1 bis 10, wobei die Homogenisierung bei 1000 U/min bis 10000 U/min durchgeführt wird.

12. Verfahren nach einem der Ansprüche 1 bis 11, wobei die mechanische Kraft für 5 Sekunden bis 20 Minuten ausgeübt wird.

13. Verfahren nach Anspruch 1, zudem umfassend einen Schritt, bei dem die aus Fettgewebe stammenden Stammzellen aus der stromalen vaskulären Fraktion, die aus dem Verfahren nach Anspruch 1 erhalten wird, isoliert werden.

## Revendications

1. Procédé de récupération d'une fraction vasculaire stromale à partir d'un échantillon, le procédé comprenant la fourniture d'une force mécanique capable de rompre l'échantillon en une suspension monocellulaire tout en maintenant les structures cellulaires des cellules de fraction vasculaire intactes, dans lequel la force mécanique est un hachage et/ou une homogénéisation, le procédé étant une digestion non enzymatique.

2. Procédé selon la revendication 1, dans lequel l'échantillon est un tissu adipeux.

3. Procédé selon la revendication 1, dans lequel le procédé comprend en outre l'étape d'isolement de la fraction vasculaire stromale à partir de la suspension monocellulaire.

4. Procédé selon la revendication 3, dans lequel l'étape d'isolement de la fraction vasculaire stromale est conduite par gradient de densité, cytométrie en flux, filtration sur membrane, adsorption sur membrane ou centrifugation.

5. Procédé selon la revendication 1, dans lequel la fraction vasculaire stromale comprend l'une quelconque des cellules suivantes : cellules souches dérivées de tissu adipeux, cellules mésenchymateuses, cellules hématopoiétiques, cellules souches / progénitrices hématopoïétiques, chondrocytes, ostéoblastes, ostéoclastes, cellules précurseurs ou progénitrices endothéliales, cellules endothéliales, cellules de muscle lisse, péricytes, cellules CD34+, cellules CD29+, cellules CD166+, cellules souches Thy-1+ ou CD90+, cellules CD44+ et cellules immunitaires choisies dans le groupe constitué des monocytes, leucocytes, lymphocytes, cellules B et T, cellules NK, macrophages, cellules dendritiques, leucocytes neutrophiles, neutrophiles, éosinophiles, basophiles, granulocytes, érythrocytes, mégacaryocytes, plaquettes.

6. Procédé selon la revendication 1, dans lequel l'échantillon est obtenu à partir d'un mammifère.

7. Procédé selon la revendication 6, dans lequel le mammifère est humain.

8. Procédé selon la revendication 1, dans lequel le procédé est conduit dans les 72 heures suivant le prélèvement de l'échantillon.

9. Procédé selon la revendication 1, le procédé étant conduit à 0 °C à 42 °C.

10. Procédé selon la revendication 1, le procédé étant conduit avec des agents qui augmentent la lipolyse de cellules adipeuses, dans lequel l'agent est choisi dans le groupe constitué de l'adrénaline, l'isobutylméthylxanthine et l'isoprénaline.

11. Procédé selon l'une quelconque des revendications 1 à 10, dans lequel l'homogénéisation est conduite à 1000 tours/min à 10 000 tours/min.

12. Procédé selon l'une quelconque des revendications 1 à 11, dans lequel la force mécanique est appliquée pendant 5 secondes à 20 minutes.

13. Procédé selon la revendication 1, comprenant en outre une étape d'isolement de cellules souches dérivées de tissu adipeux à partir de la fraction vasculaire stromale obtenue au moyen du procédé de la revendication 1.
